# EUROPEAN PATENT APPLICATION

(11) **EP 2 705 852 A1**
(43) Date of publication of application: **12.03.2014**
(21) Application number: 12183285.1
(22) Date of filing: 06.09.2012
(51) Int. Cl.: A61K 39/085

(54) **An immunogenic composition against Staphylococcus aureus comprising an inactivated recombinant non-pathogenic bacterium**

(71) Applicant: Université de Lausanne, 1011 Lausanne (CH)
(72) Inventor: Que, Yok-Ai, 1011 Lausanne (CH); Veloso, Tiago Rafael, 1012 Lausanne (CH); Moreillon, Philippe, 1000 Lausanne 26 (CH); Entenza, José, 1011 Lausanne (CH)
(74) Representative: KATZAROV S.A.

(57) **Abstract**

The present invention relates to immunogenic compositions, vaccines and antibodies for the treatment and/or prevention of infections and diseases caused by *S*. *aureus* in a subject in need thereof.

SEQUENCE LISTING

<110>  Université de Lausanne

<120>  An immunogenic composition comprising an inactivated recombinant
       non-pathogenic bacterium

<130>  18806/EP

<160>  2

<170>  PatentIn version 3.5

<210>  1
<211>  932
<212>  PRT
<213>  Staphylococcus aureus

<400>  1

Met Asn Met Lys Lys Lys Glu Lys His Ala Ile Arg Lys Lys Ser Ile
1               5                   10                  15

Gly Val Ala Ser Val Leu Val Gly Thr Leu Ile Gly Phe Gly Leu Leu
            20                  25                  30

Ser Ser Lys Glu Ala Asp Ala Ser Glu Asn Ser Val Thr Gln Ser Asp
        35                  40                  45

Ser Ala Ser Asn Glu Ser Lys Ser Asn Asp Ser Ser Ser Val Ser Ala
    50                  55                  60

Ala Pro Lys Thr Asp Asp Thr Asn Val Ser Asp Thr Lys Thr Ser Ser
65                  70                  75                  80

Asn Thr Asn Asn Gly Glu Thr Ser Val Ala Gln Asn Pro Ala Gln Gln
                85                  90                  95

Glu Thr Thr Gln Ser Ser Ser Thr Asn Ala Thr Thr Glu Glu Thr Pro
            100                 105                 110

Val Thr Gly Glu Ala Thr Thr Thr Thr Thr Asn Gln Ala Asn Thr Pro
        115                 120                 125

Ala Thr Thr Gln Ser Ser Asn Thr Asn Ala Glu Glu Leu Val Asn Gln
    130                 135                 140

Thr Ser Asn Glu Thr Thr Phe Asn Asp Thr Asn Thr Val Ser Ser Val
145                 150                 155                 160

Asn Ser Pro Gln Asn Ser Thr Asn Ala Glu Asn Val Ser Thr Thr Gln
                165                 170                 175

Asp Thr Ser Thr Glu Ala Thr Pro Ser Asn Asn Glu Ser Ala Pro Gln
            180                 185                 190

Ser Thr Asp Ala Ser Asn Lys Asp Val Val Asn Gln Ala Val Asn Thr

        195                 200                 205

Ser Ala Pro Arg Met Arg Ala Phe Ser Leu Ala Ala Val Ala Ala Asp
    210                 215                 220

Ala Pro Ala Ala Gly Thr Asp Ile Thr Asn Gln Leu Thr Asn Val Thr
225                 230                 235                 240

Val Gly Ile Asp Ser Gly Thr Thr Val Tyr Pro His Gln Ala Gly Tyr
                245                 250                 255

Val Lys Leu Asn Tyr Gly Phe Ser Val Pro Asn Ser Ala Val Lys Gly
            260                 265                 270

Asp Thr Phe Lys Ile Thr Val Pro Lys Glu Leu Asn Leu Asn Gly Val
        275                 280                 285

Thr Ser Thr Ala Lys Val Pro Pro Ile Met Ala Gly Asp Gln Val Leu
    290                 295                 300

Ala Asn Gly Val Ile Asp Ser Asp Gly Asn Val Ile Tyr Thr Phe Thr
305                 310                 315                 320

Asp Tyr Val Asn Thr Lys Asp Asp Val Lys Ala Thr Leu Thr Met Pro
                325                 330                 335

Ala Tyr Ile Asp Pro Glu Asn Val Lys Lys Thr Gly Asn Val Thr Leu
            340                 345                 350

Ala Thr Gly Ile Gly Ser Thr Thr Ala Asn Lys Thr Val Leu Val Asp
        355                 360                 365

Tyr Glu Lys Tyr Gly Lys Phe Tyr Asn Leu Ser Ile Lys Gly Thr Ile
    370                 375                 380

Asp Gln Ile Asp Lys Thr Asn Asn Thr Tyr Arg Gln Thr Ile Tyr Val
385                 390                 395                 400

Asn Pro Ser Gly Asp Asn Val Ile Ala Pro Val Leu Thr Gly Asn Leu
                405                 410                 415

Lys Pro Asn Thr Asp Ser Asn Ala Leu Ile Asp Gln Gln Asn Thr Ser
            420                 425                 430

Ile Lys Val Tyr Lys Val Asp Asn Ala Ala Asp Leu Ser Glu Ser Tyr
        435                 440                 445

Phe Val Asn Pro Glu Asn Phe Glu Asp Val Thr Asn Ser Val Asn Ile
    450                 455                 460

Thr Phe Pro Asn Pro Asn Gln Tyr Lys Val Glu Phe Asn Thr Pro Asp
465                 470                 475                 480

Asp Gln Ile Thr Thr Pro Tyr Ile Val Val Val Asn Gly His Ile Asp
                485                 490                 495

Pro Asn Ser Lys Gly Asp Leu Ala Leu Arg Ser Thr Leu Tyr Gly Tyr
            500                 505                 510

Asn Ser Asn Ile Ile Trp Arg Ser Met Ser Trp Asp Asn Glu Val Ala
        515                 520                 525

Phe Asn Asn Gly Ser Gly Ser Gly Asp Gly Ile Asp Lys Pro Val Val
    530                 535                 540

Pro Glu Gln Pro Asp Glu Pro Gly Glu Ile Glu Pro Ile Pro Glu Asp
545                 550                 555                 560

Ser Asp Ser Asp Pro Gly Ser Asp Ser Gly Ser Asp Ser Asn Ser Asp
                565                 570                 575

Ser Gly Ser Asp Ser Gly Ser Asp Ser Thr Ser Asp Ser Gly Ser Asp
            580                 585                 590

Ser Ala Ser Asp Ser Asp Ser Ala Ser Asp Ser Asp Ser Ala Ser Asp
        595                 600                 605

Ser Asp Ser Ala Ser Asp Ser Asp Ser Ala Ser Asp Ser Asp Ser Asp
    610                 615                 620

Asn Asp Ser Asp Ser Asp Ser Asp Ser Asp Ser Asp Ser Asp Ser Asp
625                 630                 635                 640

Ser Asp Ser Asp Ser Asp Ser Asp Ser Asp Ser Asp Ser Asp Ser Asp
                645                 650                 655

Ser Asp Ser Asp Ser Asp Ser Asp Ser Asp Ser Asp Ser Asp Ser Asp
            660                 665                 670

Ser Asp Ser Asp Ser Asp Ser Asp Ser Asp Ser Asp Ser Asp Ser Asp
        675                 680                 685

Ser Asp Ser Asp Ser Asp Ser Asp Ser Asp Ser Asp Ser Asp Ser Asp
    690                 695                 700

Ser Asp Ser Asp Ser Asp Ser Asp Ser Asp Ser Asp Ser Asp Ser Asp
705                 710                 715                 720

Ser Asp Ser Asp Ser Asp Ser Asp Ser Asp Ser Asp Ser Asp Ser Asp
                725                 730                 735

Ser Asp Ser Asp Ser Asp Ser Asp Ser Asp Ser Asp Ser Asp Ser Asp
            740                 745                 750

Ser Asp Ser Asp Ser Asp Ser Asp Ser Asp Ser Asp Ser Asp Ser Ala
        755                 760                 765

Ser Asp Ser Asp Ser Asp Ser Asp Ser Asp Ser Asp Ser Asp Ser Asp
    770                 775                 780

Ser Asp Ser Asp Ser Asp Ser Asp Ser Asp Ser Asp Ser Asp Ser Asp
785                 790                 795                 800

Ser Asp Ser Asp Ser Asp Ser Glu Ser Asp Ser Asp Ser Glu Ser Asp
                805                 810                 815

Ser Asp Ser Asp Ser Asp Ser Asp Ser Asp Ser Asp Ser Asp Ser Asp
            820                 825                 830

Ser Asp Ser Asp Ser Ala Ser Asp Ser Asp Ser Gly Ser Asp Ser Asp
        835                 840                 845

Ser Ser Ser Asp Ser Asp Ser Glu Ser Asp Ser Asn Ser Asp Ser Glu
    850                 855                 860

Ser Gly Ser Asn Asn Asn Val Val Pro Pro Asn Ser Pro Lys Asn Gly
865                 870                 875                 880

Thr Asn Ala Ser Asn Lys Asn Glu Ala Lys Asp Ser Lys Glu Pro Leu
                885                 890                 895

Pro Asp Thr Gly Ser Glu Asp Glu Ala Asn Thr Ser Leu Ile Trp Gly
            900                 905                 910

Leu Leu Ala Ser Ile Gly Ser Leu Leu Leu Phe Arg Arg Lys Lys Glu
        915                 920                 925

Asn Lys Asp Lys
    930

<210>  2
<211>  3499
<212>  DNA
<213>  Staphylococcus aureus

<400>  2
ggtaccataa attacacatc tgcttttgaa aaaatatgat ttcaagctag gattacatta      60

ggtagagttc atattaataa taaaaaatgt ttgcaatcaa atcgtacgtt gtcgtttgta     120

attcttaaaa tagcaataaa taaaatgttt gttagtaaag tattattgtg gataataaaa     180

tatcgataca aattaattgc tataatgcaa ttttagtgta taattccatt aacagagatt     240

aaatatatct ttaaagggta tatagttaat ataaaatgac tttttaaaaa gagggaataa     300

aatgaatatg aagaaaaaag aaaaacacgc aattcggaaa aaatcgattg gcgtggcttc     360

agtgcttgta ggtacgttaa tcggttttgg actactcagc agtaaagaag cagatgcaag     420

tgaaaatagt gttacgcaat ctgatagcgc aagtaacgaa agcaaaagta atgattcaag 480
tagcgttagt gctgcaccta aaacagacga cacaaacgtg agtgatacta aaacatcgtc 540
aaacactaat aatggcgaaa cgagtgtggc gcaaaatcca gcacaacagg aaacgacaca 600
atcatcatca acaaatgcaa ctacggaaga aacgccggta actggtgaag ctactactac 660
gacaacgaat caagctaata caccggcaac aactcaatca agcaatacaa atgcggagga 720
attagtgaat caaacaagta atgaaacgac ttttaatgat actaatacag tatcatctgt 780
aaattcacct caaaattcta caaatgcgga aaatgtttca acaacgcaag atacttcaac 840
tgaagcaaca ccttcaaaca atgaatcagc tccacagagt acagatgcaa gtaataaaga 900
tgtagttaat caagcggtta atacaagtgc gcctagaatg agagcattta gtttagcggc 960
agtagctgca gatgcaccgg cagctggcac agatattacg aatcagttga cgaatgtgac 1020
agttggtatt gactctggta cgactgtgta tccgcaccaa gcaggttatg tcaaactgaa 1080
ttatggtttt tcagtgccta attctgctgt taaaggtgac acattcaaaa taactgtacc 1140
taaagaatta aacttaaatg gtgtaacttc aactgctaaa gtgccaccaa ttatggctgg 1200
agatcaagta ttggcaaatg gtgtaatcga tagtgatggt aatgttattt atacatttac 1260
agactatgta aatactaaag atgatgtaaa agcaactttg accatgcccg cttatattga 1320
ccctgaaaat gttaaaaaga caggtaatgt gacattggct actggcatag gtagtacaac 1380
agcaaacaaa acagtattag tagattatga aaaatatggt aagttttata acttatctat 1440
taaaggtaca attgaccaaa tcgataaaac aaataatacg tatcgtcaga caatttatgt 1500
caatccaagt ggagataacg ttattgcgcc ggttttaaca ggtaatttaa aaccaaatac 1560
ggatagtaat gcattaatag atcagcaaaa tacaagtatt aaagtatata aagtagataa 1620
tgcagctgat ttatctgaaa gttactttgt gaatccagaa aactttgagg atgtcactaa 1680
tagtgtgaat attacattcc caaatccaaa tcaatataaa gtagagttta atacgcctga 1740
tgatcaaatt acaacaccgt atatagtagt tgttaatggt catattgatc cgaatagcaa 1800
aggtgattta gctttacgtt caactttata tgggtataac tcgaatataa tttggcgctc 1860
tatgtcatgg gacaacgaag tagcatttaa taacggatca ggttctggtg acggtatcga 1920
taaaccagtt gttcctgaac aacctgatga gcctggtgaa attgaaccaa ttccagagga 1980
ttcagattct gacccaggtt cagattctgg cagcgattct aattcagata gcggttcaga 2040
ttcgggtagt gattctacat cagatagtgg ttcagattca gcgagtgatt cagattcagc 2100
aagtgattca gactcagcga gtgattcaga ttcagcaagc gattccgact cagcgagcga 2160
ttccgactca gacaatgact cggattcaga tagcgattct gactcagaca gtgactcaga 2220
ttccgacagt gactcagatt cagatagcga ttctgactca gacagtgact cagattcaga 2280
tagcgattca gattcagata gcgattcaga ttccgacagt gattccgact cagacagcga 2340
ttctgactcc gacagtgatt ccgactcaga cagcgattca gattccgaca gtgattccga 2400
ctcagatagc gattccgact cagatagcga ctcagattca gacagcgatt cagattcaga 2460
cagcgattca gattcagata gcgattcaga ttccgacagt gactcagatt ccgacagtga 2520

ctcggattca gatagcgatt cagattccga cagtgactca gattccgaca gtgactcaga 2580
ctcagacagt gattcggatt cagcgagtga ttcggattca gatagtgatt ccgactccga 2640
cagtgactcg gattcagata gcgactcaga ctcggatagc gactcggatt cagatagcga 2700
ttcggactca gatagcgatt cagaatcaga cagcgattca gaatcagaca gcgattcaga 2760
ttcagacagc gactcagaca gtgactcaga ttcagatagt gactcggatt cagcgagtga 2820
ttcagactca ggtagtgact ccgattcatc aagtgattcc gactcagaaa gtgattcaaa 2880
tagcgattcc gagtcaggtt ctaacaataa tgtagttccg cctaattcac ctaaaaatgg 2940
tactaatgct tctaataaaa atgaggctaa agatagtaaa gaaccattac cagatacagg 3000
ttctgaagat gaagcaaata cgtcactaat ttggggatta ttagcatcaa taggttcatt 3060
actacttttc agaagaaaaa aagaaaataa agataagaaa taagtaataa tgatattaaa 3120
ttaatcatat gattcatgaa gaagccacct taaaaggtgc ttctttactt tggattttcc 3180
aaatatattg tttgaatata attaataatt aattcatcaa cagttaatta ttttaaaaag 3240
gtagatgtta tataatttgg cttggcgaaa aaatagggtg taaggtaggt tgttaattag 3300
ggaaaattaa ggagaaaata cagttgaaaa ataaattgct agttttatca ttgggagcat 3360
tatgtgtatc acaaatttgg gaaagtaatc gtgcgagtgc agtggtttct ggggagaaga 3420
atccatatgt atctgagtcg ttgaaactga ctaataataa aaataaatct agaacagtag 3480
aagagtataa gaaaagctt 3499

## Description

### FIELD OF THE INVENTION

The present invention relates to immunogenic compositions, vaccines and antibodies for the treatment and/or prevention of infections and diseases caused by *S*. *aureus* in a subject in need thereof.

### BACKGROUND OF THE INVENTION

*Staphylococcus aureus* is a major pathogen responsible for a variety of diseases, from benign skin infections, such as folliculitis and furunculosis, to life-threatening conditions, including erysipelas, deep-seated abscesses, osteomyelitis, pneumonia, sepsis, and infective endocarditis (IE). In addition to infections in which the organism is physically present at the infected site, *S*. *aureus* is also capable of producing "distant" diseases, which are mediated by the secretion of toxins. This success is ensured by the coordinated expression of numerous surface adhesins, which mediate host-tissue colonization, and secreted proteins and toxins, which promote invasion as well as strategies to escape the host immune system (Que et al., 2009).

For instance, *S. aureus* adhesins - which are collectively referred to as MSCRAMMs for Microbial Surface Components Reacting with Adherence Matrix Molecules 11-encompass at least 21 surface-anchored proteins including fibrinogen-binding proteins A and B (clumping factors A and B, or ClfA and ClfB), fibronectin-binding proteins A and B (FnPBA and FnBPB), collagen-binding protein (Cna) and protein A (Spa) to mention just a few (Patti et al., 1994).

Previous studies have shown that ClfA is essential for the development of IE. Moreover, after individual expression of ClfA in the non-pathogenic bacteria *Lactococcus lactis,* it has been shown that fibrinogen-binding was pivotal in promoting IE. In addition ClfA also interacts with the GPIIβIIIα receptor on the surface of platelets, a feature that plays an important indirect role in the ability of *S*. *aureus* to induce IE (Que et al., 2011).

Numerous attempts to develop vaccines against a variety of these structures, especially against the fibrinogen binding-domain of ClfA, have been attempted with various successes in animal models, but none of them have achieved sustainable efficacy in human clinical trials (Broughan et al., 2011).

In parallel, expressing antigens in non-pathogenic *L. lactis* has been attempted to trigger mucosal immunity. However, technical issues such as *in vivo* persistence of the bacterial strain as well as antigen release are as yet incompletely solved(Wells et al., 2008).

However, despite these studies and attempts, there is currently no efficacious immunogenic composition for treating and/or preventing *S. aureus* infections and diseases caused by *S*. *aureus.*

This object has been achieved by providing an immunogenic composition comprising an inactivated recombinant non-pathogenic bacterium, or a part thereof, expressing on its cell surface, at least one natively folded sequence of a *S*. *aureus* adhesin.

### SUMMARY OF THE INVENTION

The invention provides an immunogenic composition comprising an inactivated recombinant non-pathogenic bacterium, or a part thereof, expressing on its cell surface, at least one natively folded sequence of a *S*. *aureus* adhesin.

Furthermore, the invention also provides a vaccine comprising an immunogenic composition of the invention in an immunologically acceptable carrier or diluent.

The invention further provides the use of the vaccine of the invention for the treatment and/or prevention of infections and diseases caused by *S*. *aureus* in a subject in need thereof.

Also provided is an isolated and/or purified antibody, antibody fragment or derivative thereof able to bind to the at least one natively folded sequence of a *S*. *aureus* adhesin expressed on the cell surface of an inactivated recombinant non-pathogenic bacterium.

### BRIEF DESCRIPTION OF THE FIGURE

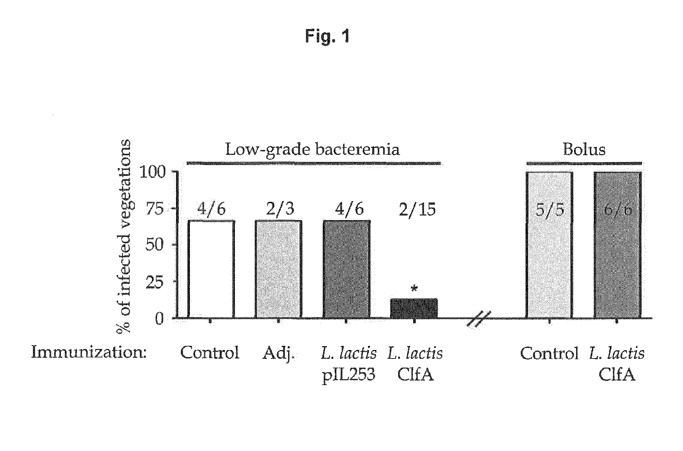

**Figure 1****. Prevention of *S. aureus* experimental endocarditis (i.e. infected vegetations) in rats immunized with vaccine preparations.** Rats were immunized with various vaccine preparations (see infra) for 6 weeks as described, before aortic vegetations were induced. 24h later, they were challenged with identical inoculum sizes (10⁴ CFU) administered either by continuous infusion (0.0017 ml/min over 10 h), or by i.v. bolus (1 ml in 1 min). * *P* < *0.05* compared to the control group by χ² test. Control: group immunized with PBS; Adj.: group immunized with Freund's adjuvant group with the adjuvant emulsified at a 1:1 ratio in PBS; *L*. *lactis* pIL253 and *L. lactis* ClfA: groups immunized with *L*. *lactis* pIL253 or ClfA, respectively, emulsified at a 1:1 ratio in Freund's adjuvant.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" or "comprising" is generally used in the sense of include/including, that is to say permitting the presence of one or more features or components.

As used in the specification and claims, the singular form "a", "an" and "the" include plural references unless the context clearly dictates otherwise.

As used herein, "at least one" means "one or more."

As used herein, the terms "protein", "polypeptide", "polypeptidic", "peptide" and "peptidic" are used interchangeably herein to designate a series of amino acid residues connected to the other by peptide bonds between the alpha-amino and carboxy groups of adjacent residues.

As used herein the term "subject" is well-recognized in the art, and, is used herein to refer to a mammal, including dog, cat, rat, mouse, monkey, cow, horse, goat, sheep, pig, camel, and, most preferably, a human. The term does not denote a particular age or sex. Thus, adult and newborn subjects, whether male or female, are intended to be covered.

The present invention provides an immunogenic composition comprising an inactivated recombinant non-pathogenic bacterium, or a part thereof, expressing on its cell surface, at least one natively folded sequence of a *S*. *aureus* adhesin.

A "non-pathogenic bacterium" refers to a bacterium that does not cause a disease state when in contact with a subject. Examples of non-pathogenic bacteria are selected from the non-limiting group comprising the Bacillus genus, Lactobacillus genus, *Lactococcus* genus, *Sporolactobacillus* genus, *Bifidobacterium* genus, and the like bacteria. Preferably, the non-pathogenic bacterium is selected from the group comprising *L*. *lactis, L. acidophilus* and *Lactobacilli.* Particularly preferred is *L*. *lactis* and most particularly prefred is *L. lactis* subpecies cremoris 1363.

*Lactococcus lactis* is a nonpathogenic bacterium that is being developed, in its live form, as a vaccine delivery vehicle for immunization by mucosal routes.

However, the non-pathogenic bacterium of the invention is in an inactivated form. Inactivation is performed using methods and/or compounds known in the art, such as for example by H₂O₂, formaldehyde, heat or UV treatments.

The present invention also refers to a part of said inactivated recombinant non-pathogenic bacterium. Usually, this consists in an isolated and/or purified cell wall of the inactivated recombinant non-pathogenic bacterium.

Generally, the non-pathogenic bacterium of the invention is a recombinant bacterium as it comprises at least one heterologous nucleic acid molecule encoding a natively folded sequence of a *S*. *aureus* adhesin.

Usually, the nucleic acid molecule encoding a natively folded sequence of a *S*. *aureus* adhesin is in the form of deoxyribonucleic acid (DNA). DNA which can be used herein is any polydeoxynuclotide sequence, including, e.g. double-stranded DNA, single-stranded DNA, double-stranded DNA wherein one or both strands are composed of two or more fragments, double-stranded DNA wherein one or both strands have an uninterrupted phosphodiester backbone, DNA containing one or more single-stranded portion(s) and one or more double-stranded portion(s), double-stranded DNA wherein the DNA strands are fully complementary, double-stranded DNA wherein the DNA strands are only partially complementary, circular DNA, covalently- closed DNA, linear DNA, covalently cross-linked DNA, cDNA, chemically- synthesized DNA, semi-synthetic DNA, biosynthetic DNA, naturally-isolated DNA, enzyme-digested DNA, sheared DNA, labeled DNA, such as radiolabeled DNA and fluorochrome-labeled DNA, DNA containing one or more non-naturally occurring species of nucleic acid.

DNA sequences that encode a natively folded sequence of a *S*. aureus adhesin can be synthesized by standard chemical techniques, for example, the phosphotriester method or via automated synthesis methods and PCR methods.

The DNA sequence encoding a natively folded sequence of a *S*. *aureus* adhesin according to the invention may also be produced by enzymatic techniques. Thus, restriction enzymes, which cleave nucleic acid molecules at predefined recognition sequences can be used to isolate nucleic acid sequences from larger nucleic acid molecules containing the nucleic acid sequence, such as DNA (or RNA) that codes for a peptide consisting a natively folded sequence of a *S*. *aureus* adhesin.

Encompassed by the present invention is also a nucleic acid in the form of a polyribonucleotide (RNA), including, e.g., single-stranded RNA, cRNA, double- stranded

RNA, double-stranded RNA wherein one or both strands are composed of two or more fragments, double-stranded RNA wherein one or both strands have an uninterrupted phosphodiester backbone, RNA containing one or more single-stranded portion(s) and one or more double-stranded portion(s), double-stranded RNA wherein the RNA strands are fully complementary, double-stranded RNA wherein the RNA strands are only partially complementary, covalently crosslinked RNA, enzyme-digested RNA, sheared RNA, mRNA, chemically-synthesized RNA, semi-synthetic RNA, biosynthetic RNA, naturally-isolated RNA, labeled RNA, such as radiolabeled RNA and fluorochrome-labeled RNA, RNA containing one or more non-naturally- occurring species of nucleic acid.

The present invention also includes variants of the aforementioned sequences that are nucleotide sequences that vary from the reference sequence by conservative nucleotide substitutions, whereby one or more nucleotides are substituted by another with same characteristics.

The invention also encompasses allelic and polymorphic variants of the aforementioned sequences; that is, naturally-occurring alternative forms of the natively folded sequence of a *S*. *aureus* adhesin that also encode peptides that are identical, homologous or related to that encoded by the sequence of a *S*. *aureus* adhesin. Alternatively, non-naturally occurring variants may be produced by mutagenesis techniques or by direct synthesis.

Preferably the acid molecule encoding a natively folded sequence of a *S*. *aureus* adhesin is a genomic DNA as set forth in **SEQ ID** N° **2.**

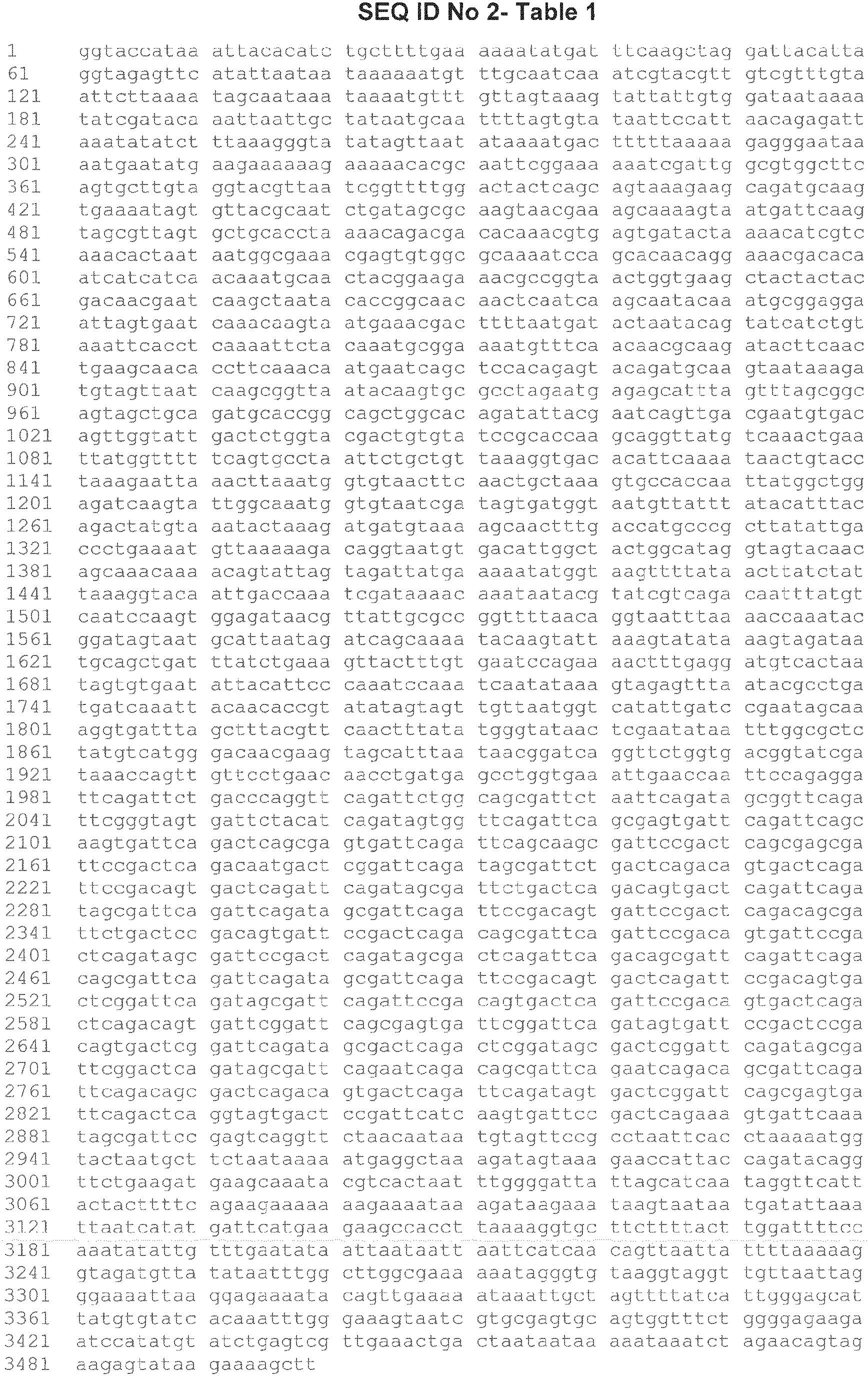

Yet another concern of the present invention is to provide an expression vector comprising at least one copy of a nucleic acid molecule encoding a natively folded sequence of a *S*. *aureus* adhesin. Preferably the nucleic acid molecule sequence encoding a peptide of the invention is DNA.

As used herein, "vector", "plasmid" and "expression vector" are used interchangeably, as the plasmid is the most commonly used vector form.

The vector may further comprise a promoter operably linked to the sequence encoding a natively folded sequence of a *S*. *aureus* adhesin. This means that the linked isolated and purified DNA sequence encoding the peptide of the present invention is under control of a suitable regulatory sequence which allows expression, i.e. transcription and translation of the inserted isolated and purified DNA sequence.

As used herein, the term "promoter" designates any additional regulatory sequences as known in the art e.g. a promoter and/or an enhancer, polyadenylation sites and splice junctions usually employed for the expression of the polypeptide or may include additionally one or more separate targeting sequences and may optionally encode a selectable marker. Promoters which can be used provided that such promoters are compatible with the host cell are e.g promoters obtained from the genomes of viruses such as polyoma virus, adenovirus (such as Adenovirus 2), papilloma virus (such as bovine papilloma virus), avian sarcoma virus, cytomegalovirus (such as murine or human cytomegalovirus immediate early promoter), a retrovirus, hepatitis-B virus, and Simian Virus 40 (such as SV 40 early and late promoters) or promoters obtained from heterologous mammalian promoters, such as the actin promoter or an immunoglobulin promoter or heat shock promoters.

Enhancers, which can be used, are e.g. enhancer sequences known from mammalian genes (globin, elastase, albumin, a-fetoprotein, and insulin) or enhancer from a eukaryotic cell virus. e.g. the SV40 enhancer, the cytomegalovirus early promoter enhancer, the polyoma, and adenovirus enhancers.

Useful expression vectors, for example, may consist of segments of chromosomal, non-chromosomal and synthetic DNA sequences. Suitable vectors include derivatives of SV40 and known bacterial plasmids, phage DNAs, yeast plasmids such as the 2µ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like. Most preferably the expression vector is a lactococcal plasmid. More preferably, the lactococcal plasmid is pOri23.

In a preferred embodiment, the sequence of the *S*. *aureus* adhesin of the invention is a natively folded sequence and is as set forth in SEQ ID No 1 below.

A "natively folded sequence" refers to a protein that is structured under physiologic conditions. Preferably, the sequence of the invention consists in a whole functional amino acid sequence (amino acid 1 to 932, table 1), rather than in a partial or an incomplete sequence.

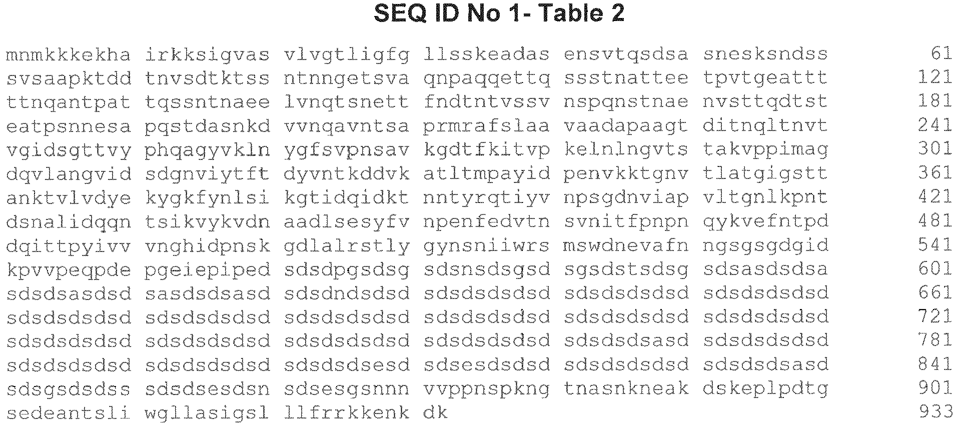

Preferably, the *S*. *aureus* adhesin is selected from the group comprising fibrinogen-binding protein A (clumping factor A ClfA), fibrinogen-binding protein B (ClfB), fibronectin-binding protein A (FnPBA) and fibronectin-binding protein B (FnBPB), collagen-binding protein (Cna) and protein A (Spa), Serine-aspartate repeat protein C, D and E (SdrC-E), Plasmin-sensitive protein (Pls), Factor affecting methicillin resistance in the presence of Triton X-100 (FmtB), surface protein A - K (SasA-K).

Most preferably, the *S*. *aureus* adhesin is the fibrinogen-binding protein A (clumping factor A (ClfA)). Clumping factor A (ClfA) is an MSCRAMM protein expressed by *S*. *aureus* that promotes binding of fibrinogen and fibrin to the bacterial cell surface. ClfA is the prototype of a recently identified multigene family of cell surface proteins characterized by a common domain composed of a unique serine-aspartate repeat. The gene encoding the fibrinogen-binding protein shows a 933-amino-acid polypeptide that contains structural features characteristic of many cell surface-associated proteins from gram-positive bacteria, including a typical cell wall attachment region comprising an LPXTG motif, a hydrophobic transmembrane sequence, and a positively charged C terminus. The fibrinogen-binding domain of ClfA has been localized to a 218-residue segment within region A.

Surprisingly, the Applicants of the present invention have successfully prevented *S. aureus* experimental endocarditis in rats vaccinated with UV-killed *L. lactis* expressing heterologously the staphylococcal adhesin ClfA. The success of vaccination is probably due to the method of antigen delivery, i.e. a whole functional *S*. *aureus* surface adhesin on a genuine bacterial surface, rather than only restricted peptides of the protein. This mode of delivery is likely to largely widen the repertoire of anti-staphylococcal antibodies generated by the host, and thus increase the efficacy of protection.

The cell wall anchored fibrinogen-binding protein ClfA has been the major target of vaccine candidates, due to his ability to binds to the γ-chain of the fibrinogen. Indeed, previous studies have shown that ClfA is essential for the development of IE due to the pivotal role of fibrinogen-binding(Moreillon et al., 1995; Yok-ai Que et al., 2005). However, numerous attempts to develop vaccines against a variety of *S*. *aureus* virulence factors have been attempted with various successes in animal models, but have as yet not achieved sustainable efficacy in human clinical trials.

The reasons for these failures are not entirely clarified, but there are at least two parameters of the *S*. *aureus* camouflage system that might have been underestimated until now. First, the fact that the bacterium may vary the way it exposes its antigenic structure on the surface, and second the fact that different strains may undergo antigenic variations. As a result, vaccination against one particular structure that is valid against one strain may not be efficacious against another strain. Therefore, a blocking or opsonizing antibody that is active in certain circumstances may become inactive in other settings.

Staphylococcal adhesins of the LPXTG-protein family are equipped with a spacer domain between the cell wall anchor and the outermost binding domain. This spacer is important to expose the binding domain on top of the plethora of other constituents of the staphylococcal envelope, in order to bind to the target host tissue. Thus, depending on the length of this spacer (which may vary between strains) and the size of other surface components (for instance polysaccharides and protein A), the binding domain of the adhesin may become embedded in other surface structures and hidden to the immune system (Scarpa *et al.,* 2010). Indeed, it was shown that *S. aureus* exposed differently the fibrinogen-binding protein domain "A" of ClfA (Mcdevitt et al., 1994), as well as the surface capsule, at various stages of *in vivo* infection (Risley et al., 2007).

Another example comes from the length of anti-phagocytic protein A, which binds antibodies by their Fc fragment. It was recently shown that a longer spacer sequence allowed protein A to better prevent binding of antibodies to various antigenic structures presented on the staphylococcal surface (Scarpa *et al.,* 2010). Moreover, the polymorphism of the protein A gene may affect the efficacy of vaccines in a strain-dependent manner. Indeed, the length of the spacer is determined by series of repeats that are known to vary between different staphylococcal strains, and thus are currently used as phylogenic markers (Kuhn et al., 2007).

Eventually, *S. aureus* produces a plethora of toxins and superantigens that may interfere with the immune response, and thus help the organism to circumvent existing host immune strategies. Vaccination against such structures were also recently attempted (Broughan *et al.,* 2011).

The importance of antigenic variation in *S. aureus* is less clear. Indeed, only two major capsular types (types 5 and 8) are implicated in infection in human, and surface proteins and toxins are relatively well conserved (Shinefield *et al.,* 2002). However, many gram-positive pathogens, including group A streptococci and *Streptococcus pneumoniae,* undergo wide genetic variability at the level of the anti-phagocytic M protein and the polysaccharidic capsule, respectively. Taken together, the current consensus for anti-S. *aureus* vaccines is that they should comprise several antigens in order to be effective, and that single antigen-based vaccines are bound to fail (Broughan *et al.,* 2011). However, the ideal antigen mixture to be used has yet to be elucidated.

Examples showed hereafter that *S*. *aureus* adhesins could be expressed functionally in *L. Lactis in vitro,* and could promote experimental endocarditis by the recombinant lactococci i*n vivo.* When conjugated with the proteomic dissection of the surface proteome of *S*. *aureus,* it appeared that the *S*. *aureus* proteins expressed on the surface of lactococci were entirely accessible to trypsin digestion for analysis by LC-MS (liquid chromatography coupled with mass spectrometry) (Ythier *et al.,* 2012). In contrast, this was not the case in *S*. *aureus,* where trypsin had a limited access to the surface proteins, indicating that part of their structure was embedded in the wall and inaccessible to digestion, and thus to recognition by antibodies as well. Since all these domains were variously exposed on the *S*. *aureus* surface, it was conceivable that vaccination against only one binding-domain, which might become hidden in certain circumstances, might be less effective than vaccination against the whole protein presented in a functional conformation on the surface of lactococci.

As shown in the examples, immunizing series of rats with UV-killed lactococci expressing *S*. *aureus* ClfA protected animals from subsequent experimental endocarditis due to *S*. *aureus* induced by low-grade bacteremia (*P* < 0.05 when compared to the several control groups). Remarkably, when the same vaccination schedule was tested in animals where the experimental endocarditis due to *S*. *aureus* was induced by high-grade bacteremia (traditional bolus infection) the infection rates increased (2/15 vs 6/6) and the protective effect was lost.

Attempts to vaccinate against severe infections in animal models are usually biased by the fact that most protocols administer very large bacterial inocula, in order to ensure that all untreated control animals will become infected. However, such large inocula - often in the range of 10s of million bacteria injected intravenously - are incommensurably larger than inoculum sized expected during "natural" infection in human. Thus, it is possible that such large inocula may overwhelm the immune system, and falsely underestimate the efficacy of preventive or therapeutic strategies that would otherwise be efficacious. The Applicants recently showed that this was realistic in the model of experimental endocarditis, and that challenging animals with continuous low-grade bacteremia was as infectious that transient high-grade inoculation, but represented much more the reality of the disease in human (Veloso *et al.,* 2011). Importantly, the good results of the vaccination strategy reported above were achieved with this very realistic model. Indeed, vaccination was less effective against the standard high-grade bacteremia model, an observation that could also explain failures with other types of potential anti-endocarditis vaccines tested in animals in the past, and abandoned.

The present invention also concerns a vaccine. Preferably, the vaccine comprises an immunogenic composition of the invention in an immunologically acceptable carrier or diluent, for treating and/or preventing infections and diseases caused by *S*. *aureus*

The vaccine of the invention may also contain several antibodies or antibody fragments, e.g. two or three antibodies or antibody fragments that recognize(s) at least one natively folded sequence of a *S*. *aureus* adhesin expressed on the cell surface of an inactivated recombinant non-pathogenic bacterium.

The immunologically acceptable carrier is usually selected from the group comprising polysaccharide materials forming hydrogels and vesicular carriers. Preferably, the vesicular carriers are selected from the group comprising liposomes, niosomes, transfersomes, and ethosomes.

Hydrogels envisioned as immunologically acceptable carrier as know in the art and are particularly adapted for mucosal, topical, oral or injectable delivery.

The vaccines of the invention may further comprise one or more adjuvant. Adjuvants can include, but are not limited to, MPL + TDM + CWS (SIGMA), MF59 (an oil-in-water emulsion that includes 5% squalene, 0.5% sorbitan monoleate and 0.5% sorbitan trioleate Chiron), Heat-labile toxin (HLT), CRMig (nontoxic genetic mutant of diphtheria toxin), Squalene (IDEC PHARMACEUTICALS CORP. ), Ovalbumin (SIGMA), Quil A (SARGEANT, INC.), Aluminum phosphate gel (SUPERFOS BIOSECTOR), Cholera holotoxin (CT LIST BIOLOGICAL LAB. ), Cholera toxin B subunit (CTB), Cholera toxin A subunit- Protein A D-fragment fusion protein, Muramyl dipeptide (MDP), Adjumera (polyphosphazene, VIRUS RESEARCH INSTITUTE), Montanide ISA 720, SPT (an emulsion of 5% squalene, 0.2% Tween 80, 1.25% Pluronic L121 with phosphate-buffered saline ph 7.4), Avridine (M6 PHARMACEUTICALS), Bay R1005 (BAYER), Calcitrol (SIGMA), Calcium phosphate gel (SARGEANT INC. ), CRL 1005 (Block co-polymer P1205, VAXCEL CORP.), DHEA (MERCK), DMPC (GENZYME PHARMACEUTICALS and FINE CHEMICALS). DMPG (GENZYME PHARMACEUTICALS and FINE CHEMICALS), Gamma Inulin, Gerbu Adjuvant (CC BIOTECH CORP. ), GM-CSF, (IMMUNE CORP. ), GMDP (PEPTECH LIMITED), Imiquimod (3M PHARMACEUTICALS), ImmTher (ENDOREX CORPORATION), ISCOMTM (ISCOTEC AB), Iscoprep 7.0. 3 TM (ISCOTEC AB), Loxoribine, LT-Oral Adjuvant (*E. coli* labile enterotoxin, protoxin, BERNA PRODUCTS CORP. ), MTP-PE (CIBA-GEIGY LTD), Murametide, (VACSYN S. A.), Murapalmitine (VACSYN S. A.), Pluronic L121 (IDEC PHARMACEUTICALS CORP.), PMMA (INSTITUT FUR PHARMAZEUTISCHE TECHNOLOGIE), SAF-1 (SYNTEX ADJUVANT FORMULATION CHIRON), Stearyl tyrosine (BIOCHEM THERAPEUTIC INC.), Theramidea (IMMUNO THERAPEUTICS INC.), Threonyl- MDP (CHIRON), FREUNDS adjuvant (complete or incomplete), aluminum hydroxide, dimethyldioctadecyl-ammonium bromide, Adjuvax (ALPHA- BETA TECHNOLOGY), Inject Alum (PIERCE), Monophosphoryl Lipid A (RIBI IMMUNOCHEM RESEARCH), MPL+TDM (RIBI IMMUNOCHEM RESEARCH), Titermax (CYTRX), QS21, t Ribi Adjuvant System, TiterMaxGold, QS21, Adjumer, Calcitrol, CTB, LT (*E*. *coli* toxin), LPS (lipopolysaccharide), Avridine, the CpG sequences (Singh *et al.,* 1999 Singh, M. and Hagum, D., Nature Biotechnology 1999 17: 1075-81) toxins, toxoids, glycoproteins, lipids, glycolipids, bacterial cell walls, subunits (bacterial or viral), carbohydrate moieties (mono-, di , tri-, tetra-, oligo-and polysaccharide), or saponins. Combinations of various adjuvants may be used with the antigen to prepare the immunogen formulations. Adjuvants administered parentally or for the induction of mucosal immunity may also be used.

The present invention further contemplates an isolated and/or purified antibody, antibody fragment or derivative thereof able to bind to the at least one natively folded sequence of a *S*. *aureus* adhesin expressed on the cell surface of an inactivated recombinant non-pathogenic bacterium.

As used herein, an "antibody" is a protein molecule that reacts with a specific antigenic determinant or epitope and belongs to one or five distinct classes based on structural properties: IgA, IgD, IgE, IgG and IgM. The antibody may be a polyclonal (e.g. a polyclonal serum) or a monoclonal antibody, including but not limited to fully assembled antibody, single chain antibody, antibody fragment, and chimeric antibody, humanized antibody as long as these molecules are still biologically active and still bind to one natively folded sequence of a *S*. *aureus* adhesin of the invention. Preferably the antibody is a monoclonal antibody. Preferably also the monoclonal antibody will be selected from the group comprising the IgG1, IgG2, IgG2a, IgG2b, IgG3 and IgG4 or a combination thereof. Most preferably, the monoclonal antibody is selected from the group comprising the IgGI, IgG2, IgG2a, and IgG2b, or a combination thereof.

A typical antibody is composed of two immunoglobulin (Ig) heavy chains and two Ig light chains. Several different types of heavy chain exist that define the class or isotype of an antibody. These heavy chain types vary between different animals. All heavy chains contain a series of immunoglobulin domains, usually with one variable (VH) domain that is important for binding antigen and several constant (CH) domains. Each light chain is composed of two tandem immunoglobulin domains: one constant (CL) domain and one variable domain (VL) that is important for antigen binding.

The term "isolated", when used as a modifier of an antibody of the invention means that the antibody is made by the hand of man or is separated, completely or at least in part, from their naturally occurring *in vivo* environment. Generally, isolated antibodies are substantially free of one or more materials with which they normally associate with in nature, for example, one or more protein. The term "isolated" does not exclude alternative physical forms of the antibodies, such as multimers/oligomers, modifications (e g , phosphorylation, glycosylation, lipidation) or derivatized forms, or forms expressed in host cells produced by the hand of man

An "isolated" antibody can also be "substantially pure" or "purified" when free of most or all of the materials with which it typically associates with in nature. Thus, an isolated antibody that also is substantially pure or purified does not include polypeptides or polynucleotides present among millions of other sequences, such as antibodies of an antibody library or nucleic acids in a genomic or cDNA library.

Antibodies used in the present invention are not limited to whole antibody molecules and may be antibody fragments or derivatives as long as they are able to bind to the at least one natively folded sequence of a *S*. *aureus* adhesin expressed on the cell surface of an inactivated recombinant non-pathogenic bacterium and that they specifically recognize said natively folded sequence of a *S*. *aureus* adhesin.

Examples of isolated and/or purified antibody fragment or derivative thereof are selected amongst the group comprising a Fab-fragment, a F(ab2)'-fragment, a single-chain antibody, a chimeric antibody, a CDR-grafted antibody, a bivalent antibody-construct, a humanized antibody, a synthetic antibody, a chemically modified derivative thereof, a multispecific antibody, a diabody, a scFv-fragment; a dsFv-fragment, a labeled antibody, or another type of recombinant antibody. Specifically, an antibody fragment is synthesized by treating the antibody with an enzyme such as papain or pepsin, or genes encoding these antibody fragments are constructed, and expressed by appropriate host cells as known to the skilled artisan.

In a further aspect, there is provided a method for treating and/or preventing infections and diseases caused by *S*. *aureus,* in a subject in need thereof, comprising administering a pharmaceutically effective amount of an immunogenic composition according to the invention.

In a further aspect, there is provided a method for treating and/or preventing an APP-associated disease, in a subject in need thereof, comprising administering a pharmaceutically effective amount of an immunogenic composition according to the invention.

Usually, infections and diseases caused by *S*. *aureus* are selected among the group comprising IE, intravascular and intravascular device infections, bloodstream infections, deep-seated abscesses, osteomyelitis, infection of prosthetic materials, and skin and soft tissue infections.

Also envisioned is a method of inducing active immunity against a *S*. *aureus* infection in a subject in need thereof, comprising administering to said subject in need thereof i) an immunogenic composition comprising an inactivated recombinant non-pathogenic bacterium, or a part thereof, expressing on its cell surface, at least one natively folded sequence of a *S*. *aureus* adhesin or ii) a vaccine of the invention.

Also encompassed in the present invention is a method of inducing passive immunity against a *S*. aureus infection in a subject in need thereof, comprising administering to said subject in need thereof an isolated and/or purified antibody, antibody fragment or derivative thereof of the invention.

### EXAMPLES

### Example 1 - Materials and methods

### 1.1 Plasmid constructs and Bacterial strains

The immunization protocol in this study was done using the recombinant strain of the non-pathogenic *L. lactis* subsp. cremoris 1363 expressing individual *S*. *aureus* ClfA, described elsewhere (Piroth et al., 2008; Que et al., 2000; Que et al., 2001). The *S*. *aureus* Newman ClfA gene was inserted in the lactococcal plasmid pOri23 together with an erythromycin resistance determinant as described by Que *et al* (Que et al., 2000). *L. lactis* pIL253, containing the lactococcal plasmid pOri23 expressing only the erythromycin resistance determinant, and expressing no pathogenic factors, was used as the control mutant strain. All lactococci were grown at 30°C without shaking in M17 medium (Oxoid) or on M17 agar plates supplemented with 0.5% glucose and 5µg/ml erythromycin.

The well-described *S*. *aureus* strain Newman (i.e. methicillin-susceptible *S*. *aureus*) (Entenza et al., 2001) was used in the animal model of endocarditis. The *S*. *aureus* bacterial isolate was grown at 37°C in tryptic soy broth (Difco).

All the bacterial stocks were kept at -80°C in liquid medium supplemented with 20% (vol/vol) of glycerol.

### 1.2 Immunization protocol

(i) Animals. Four to six-week females (100g of weight) Wistar Han rats were purchased from Charles River, France. The rats were supplied with water and food ad libitum, and randomly allocated to **4** treatment groups as follows: (a) Control, (b) Freund's adjuvant (Sigma), (c) *L. lactis* pIL253 and (d) *L. lactis* ClfA. All animal experiments were carried out according to Swiss regulations (authorization 879.8).
(ii) Preparation of bacterial vaccine. The inactivated *L. lactis* vaccine was prepared as follows. *L. lactis* strains carrying either the empty plasmid pOri23, or the same plasmid expressing ClfA were cultured overnight at 30°C without shaking in M17 medium (Oxoid), harvested by centrifugation, resuspended in sterilized PBS, and adjusted to 1 x 10⁸ CFU/ml. The bacteria were then inactivated during 15 min under U.V. Then, the bacteria were emulsified at a 1:1 ratio in Freund's adjuvant. The first immunization was done with Freund's complete adjuvant, and the subsequent with Freund's incomplete adjuvant. The control group was immunized with PBS, and the Freund's adjuvant group with the adjuvant emulsified at a 1:1 ratio in PBS.
(iii) Vaccination schedule. The rats were immunized at 2-week intervals (days 0, 14 and 28). One hundred microlitres of the preparations were injected intraperitoneally to the respective groups. Blood samples were collected on days 7, 21 and 35; and the sera were harvested and stored at -80°C to posterior in vitro analysis (Gong et al., 2010).

### 1.3. Animal model of endocarditis

Catheter-induced aortic vegetations were produced according to the method of Heraief *et al* (Héraïef et al., 1982) Insertion of an intravenous (i.v.) line in the jugular vein and connection to a programmable infusion pump (Pump 44; Harvard Apparatus, Inc., South Natick, Mass.) to deliver the inocula was performed as described (Fluckiger *et al.,* 1994; Pea *et al.,* 2011) on the day 40 of the vaccination schedule. Bacterial inocula were prepared from overnight cultures. Microorganisms were recovered by centrifugation, washed and adjusted to the desired inoculum size in saline. The inoculum size was confirmed by colony counts on blood agar plates. Animals were inoculated 24 h after catheterization, via the infusion pump, with 1 ml of 10⁴ CFU progressively delivered at a pace of 0.0017 ml/min over 10 h in order to produce a low-grade of bacteremia(Pea et *al.,* 2011).

The traditional i.v, bolus inoculation (10⁴ CFU/ml) provoking transient high-grade bacteremia was also performed. Rats were sacrificed 24 h after the end of inoculation. Quantitative valve cultures were performed as previously described (Fluckiger *et al.,* 1994) This method permitted the detection of 2 log10 CFU/g of vegetation.

### 1.4. Statistical analysis

Statistical analyses were performed using GraphPad software (GraphPad Software, Inc., USA). The rates of valve infections of the various groups were compared by the χ2 test. *P*< *0.05* was considered to be statistically significant.

### Example 2 - Results

The efficacy of the immunization with *L. lactis* ClfA against the *S*. *aureus* induced experimental endocarditis due to low-grade bacteremia was compared to the different control groups. The results of the infectivity rate are shown in Figure 1.

The proportion of infection in the group immunized with *L. lactis* ClfA (2/15; 13.3%) was significantly lower than in the control groups PBS (4/6; 66.7%), Adj. (2/3; 66.7%) and *L*. *lactis* pIL253 (4/6; 66.7%) (χ2 test; P = 0.0347), in the case of low-grade bacteremia experimental endocarditis induced by *S*. *aureus* Newman. These results confirm the protective effect of the immunization using the non-pathogenic *L. lactis* expressing heterologously the staphylococcal adhesin ClfA. In contrast, when the same immunization schedule was used in animals exposed to high-grade bacteremia experimental endocarditis, the protective effect was not observed.

### REFERENCES

Broughan, J., Anderson, R., & Anderson, A. S. (2011). Strategies for and advances in the development of Staphylococcus aureus prophylactic vaccines. Expert review of vaccines, 10(5), 695-708.

Entenza, J.M., Que, Y. A., Vouillamoz, J., Glauser, M.P., & Moreillon, P. (2001). Efficacies of moxifloxacin, ciprofloxacin, and vancomycin against experimental endocarditis due to methicillin-resistant Staphylococcus aureus expressing various degrees of ciprofloxacin resistance. Antimicrobial agents and chemotherapy, 45(11), 3076-83.

Fluckiger, U., Moreillon, P., Blaser, L. J., Bickle, M., Glauser, M.P., & Francioli, P. (1994). Simulation of Amoxicillin Pharmacokinetics in Humans for the Prevention of Streptococcal Endocarditis in Rats. Antimicrobial agents and chemotherapy, 38(12), 2846-2849.

Gong, R., Hu, C., Xu, H., Guo, A., Chen, H., Zhang, G., & Shi, L. (2010). Evaluation of clumping factor A binding region A in a subunit vaccine against Staphylococcus aureus-induced mastitis in mice. Clinical and vaccine immunology : CVI, 17(11), 1746-52.

Héraïef, E., Glauser, M.P., & Freedman, L. R. (1982). Natural history of aortic valve endocarditis in rats. Infection and immunity, 37(1), 127-31.

Kuhn, G., Francioli, P., & Blanc, D. S. (2007). Double-locus sequence typing using clfB and spa, a fast and simple method for epidemiological typing of methicillin-resistant Staphylococcus aureus. Journal of clinical microbiology, 45(1), 54-62.

Mcdevitt, D., Francois, P. F., Vaudaux, P., & Foster, T. J. (1994). Molecular characterization of the clumping factor (fibrinogen receptor) of Staphylococcus aureus. Mol. Microbiol, 11(2), 237-248.

Moreillon, P., Entenza, J.M., Francioli, P., McDevitt, D., Foster, T. J., & Vaudaux, P. (1995). Role of Staphylococcus aureus Coagulase and Clumping Factor in Pathogenesis of Experimental Endocarditis. Infection and immunity, 63(12), 4738-4743.

Patti, J.M., Allen, B. L., Mcgavin, M. J., & Hook, M. (1994). MSCRAMM -Mediated Adherence of Microorganisms to Host Tissues. Ann. Rev. Microbiol., 48, 585-617.

Pea, F., Cojutti, P., Petrosillo, N., Furlanut, M., Entenza, J.M., Veloso, T.R., Vouillamoz, J., et al. (2011). Continuous infusion may improve the efficacy of vancomycin in treatment of experimental endocarditis due to heterogeneous vancomycin-intermediate Staphylococcus aureus. Antimicrobial agents and chemotherapy, 55(9), 4496;

Piroth, L., Que, Y., Widmer, E., Panchaud, A., & Moreillon, P. (2008). The Fibrinogen- and Fibronectin-Binding Domains of Staphylococcus aureus Fibronectin-Binding Protein A Synergistically Promote Endothelial Invasion and Experimental Endocarditis. Infection and immunity, 76(8), 3824-3831.

Que, Y, & Moreillon, P. (2011). Infective endocarditis. The Lancet, 363, 139-149. Nature Publishing Group.

Que, Y. A., Haefliger, J.A., Francioli, P., & Moreillon, P. (2000). Expression of Staphylococcus aureus clumping factor A in Lactococcus lactis subsp. cremoris using a new shuttle vector. Infection and immunity, 68(6), 3516-22.

Que, Y. A., & Moreillon, P. (2009). Principles and Practice in Infectious Diseases. (G. Mandell, R. Douglas, & J. Bennett, Eds.) (p. Chapter 195). New York: Elsevier Inc.

Que, Yok-Ai, Franc, P., Haefliger, J., Vaudaux, P., & Moreillon, P. (2001). Reassessing the Role of Staphylococcus aureus Clumping Factor and Fibronectin-Binding Protein by Expression in Lactococcus lactis. Infection and immunity, 69(10), 6296-6302.

Que, Yok-Ai, Haefliger, J., Piroth, L., Widmer, E., Sinha, B., Herrmann, M., Francioli, P., et al. (2005). Fibrinogen and fibronectin binding cooperate for valve infection and invasion in Staphylococcus aureus experimental endocarditis. JEM, 201(610), 1627-1635.

Risley, A. L., Loughman, A., Cywes-Bentley, C., Foster, T. J., & Lee, J. C. (2007). Capsular polysaccharide masks clumping factor A-mediated adherence of Staphylococcus aureus to fibrinogen and platelets. The Journal of infectious diseases, 196(6), 919-27.

Scarpa, M., Piccinini, R., Brun, P., Grillo, A., Palù, G., Mengoli, C., Daprà, V., et al. (2010). Relationship between virulence factor genes in bovine Staphylococcus aureus subclinical mastitis isolates and binding to anti-adhesin antibodies. The Journal of dairy research, 77(2), 159-67.

Shinefield, H., Black, S., Fattom, A., Horwith, G., Rasgon, S., Ordonez, J., Yeoh, H., et al. (2002). Use of a Staphylococcus aureus conjugate vaccine in patients receiving hemodialysis. The New England journal of medicine, 346(7), 491-6.

Veloso, T.R., Amiguet, M., Rousson, V., Giddey, M., Vouillamoz, J., Moreillon, P., & Entenza, J. M. (2011). Induction of experimental endocarditis by continuous low-grade bacteremia mimicking spontaneous bacteremia in humans. Infection and immunity, 79(5), 2006-11.

Wells, J.M., & Mercenier, A. (2008). Mucosal delivery of therapeutic and prophylactic molecules using lactic acid bacteria. Nature reviews. Microbiology, 6(5), 349-62.

Ythier, M., Resch, G., Waridel, P., Panchaud, A., Gfeller, A., Majcherczyk, P., Quadroni, M., et al. (2012). Proteomic and transcriptomic profiling of Staphylococcus aureus surface LPXTG-proteins: correlation with agr genotypes and adherence phenotypes. Molecular & cellular proteomics : MCP*.*

## Claims

1. An immunogenic composition comprising an inactivated recombinant non-pathogenic bacterium, or a part thereof, expressing on its cell surface, at least one natively folded sequence of a *S*. *aureus* adhesin.

2. The immunogenic composition of claim 1, wherein the part of the inactivated recombinant non-pathogenic bacterium consists in an isolated and/or purified cell wall.

3. The immunogenic composition of claim 1 or 2, wherein the *S*. *aureus* adhesin is selected from the group comprising fibrinogen-binding protein A (clumping factor A ClfA), fibrinogen-binding protein B (ClfB), fibronectin-binding protein A (FnPBA) and fibronectin-binding protein B (FnBPB), collagen-binding protein (Cna) and protein A (Spa), Serine-aspartate repeat protein C, D and E (SdrC-E), Plasmin-sensitive protein (Pls), Factor affecting methicillin resistance in the presence of Triton X-100 (FmtB), surface protein A - K (SasA-K)

4. The immunogenic composition of claim 3, wherein the *S*. *aureus* adhesin is the fibrinogen-binding protein A (clumping factor A (ClfA)).

5. The immunogenic composition of any one of the preceding claims, wherein the inactivated recombinant non-pathogenic bacterium is the *L. lactis* subspecies cremoris 1363.

6. The immunogenic composition of claim 4, wherein the sequence of *S*. *aureus* fibrinogen-binding protein A (clumping factor A (ClfA)) is as set forth in SEQ ID No.1.

7. The immunogenic composition of any one of the preceding claims, wherein the inactivated recombinant non-pathogenic bacterium is UV inactivated.

8. A vaccine comprising an immunogenic composition of any one of claims 1-7 in an immunologically acceptable carrier or diluent.

9. The vaccine of claim 8, wherein the immunologically acceptable carrier is selected from the group comprising polysaccharide materials forming hydrogels and vesicular carriers.

10. The vaccine of claim 9, wherein the vesicular carriers are selected from the group comprising liposomes, niosomes, transfersomes, and ethosomes.

11. The vaccine of any one of claims 8 to 10, further comprising an adjuvant.

12. Use of the vaccine of any one of claims 8 to 11 for the treatment and/or prevention of infections and diseases caused by *S*. *aureus* in a subject in need thereof.

13. The use of claim 12, wherein the infections and diseases caused by *S*. *aureus* is selected form the group comprising IE, intravascular and intravascular device infections, bloodstream infections, deep-seated abscesses, osteomyelitis, infection of prosthetic materials, and skin and soft tissue infections.

14. An isolated and/or purified antibody, antibody fragment or derivative thereof able to bind to the at least one natively folded sequence of a *S*. *aureus* adhesin expressed on the cell surface of an inactivated recombinant non-pathogenic bacterium.
